(19) Europäisches Patentamt
European Patent Office
Office européen des brevets

(11) Veröffentlichungsnummer: **0 332 030 A2**

(12) # EUROPÄISCHE PATENTANMELDUNG

(21) Anmeldenummer: **89103541.2**

(22) Anmeldetag: **01.03.89**

(51) Int. Cl.[4]: **C07D 213/73 , C07D 213/61 , C07D 213/69**

(30) Priorität: **11.03.88 DE 3808115**

(43) Veröffentlichungstag der Anmeldung:
**13.09.89 Patentblatt 89/37**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Bonse, Gerhard, Dr.**
**Wolfskaul 3**
**D-5000 Köln 80(DE)**
Erfinder: **Hallenbach, Werner, Dr.**
**Kleiststrasse 10**
**D-4018 Langenfeld(DE)**
Erfinder: **Lindel, Hans, Dr.**
**Carl-Duisberg-Strasse 321**
**D-5090 Leverkusen 1(DE)**

(54) **Verfahren zur Herstellung von 6-Acetyl-3-amino-2,4-dihalogen-pyridin.**

(57) Die vorliegende Erfindung betrifft ein Verfahren zur Herstellung von 6-Acetyl-3-amino-2,4-dihalogenpyridinen der Formel I

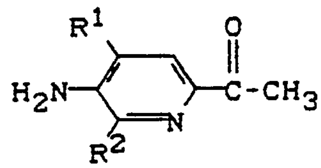

I

in welcher
$R^1$ und $R^2$ für Halogen stehen
das dadurch gekennzeichnet ist, daß man 6-Ethyl-3-amino-2,4-dihalogenpyridin der Formel II

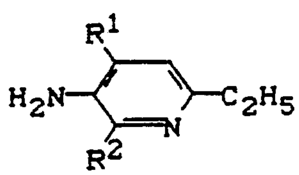

II

zunächst mit Acylierungsmitteln umsetzt, anschließend die an der Aminogruppe acylierte Verbindung der Formel II mit Kaliumpermanganat in einem bei pH-Werten zwischen 4 und 10 gehaltenen Medium oxidiert und danach den Acylrest von der Aminogruppe entfernt.

EP 0 332 030 A2

## Verfahren zur Herstellung von 6-Acetyl-3-amino-2,4-dihalogen-pyridin

Die vorliegende Erfindung betrifft ein Verfahren zur Herstellung von 6-Acetyl-3-amino-2,4-dihalogenpyridinen und Zwischenprodukte zu ihrer Herstellung.

6-Acetyl-3-amino-2,4-dihalogenpyridine sind bekannt aus (DE-OS 3 615 293). Sie werden nach dem dort angegebenen Verfahren durch Verseifung und Decarboxylierung der entsprechenden Pyridylcarbonylessigsäureestern hergestellt. Diese werden aus den entsprechenden Pyridylcarbonsäurealkylestern oder -säurechloriden durch Umsetzung mit Essigestern und einer geeigneten Base erhalten. Die Pyridylcarbonsäurealkylester bzw. -säurechloride werden aus den entsprechende Pyridylcarbonsäuren hergestellt.

Die Pyridylcarbonsäuren werden durch Oxidation des 6-Methyl-3-amino-2,4-dihalogenpyridin erhalten. 6-Methyl-3-amino-2,4-dihalogenpyridine sind bekannt. Bei dieser Reaktionsfolge ist noch nicht berücksichtigt, daß z.B. ein Aminosubstituent des Pyridylringes vor Durchführung der Reaktion an einem anderen Substituenten mit Hilfe einer Acetylierung geschützt werden muß.

Das heißt, um von 6-Methyl-3-amino-2,4-dihalogenpyridin zu 6-Acetyl-3-amino-2,4-dihalogenpyridin zu gelangen sind ohne Berücksichtigung der Einführung und des Abspaltens von Schutzgruppen am Aminosubstituent mindestens 4 Stufen erforderlich. Es bestand daher Interesse an einer einfacher durchzuführenden Synthese für 6-Acetyl-3-amino-2,4-dihalogenpyridine.

Die vorliegende Erfindung betrifft:

1. Verfahren zur Herstellung von 6-Acetyl-3-amino-2,4-dihalogenpyridinen der Formel I

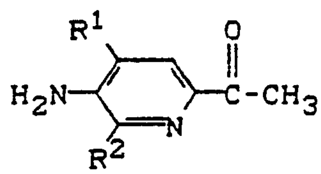

I

in welcher
$R^1$ und $R^2$ für Halogen stehen
dadurch gekennzeichnet, daß man 6-Ethyl-3-amino-2,4-dihalogenpyridin der Formel II

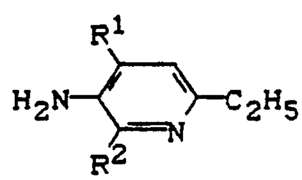

II

in welcher
$R^1$ und $R^2$ die oben angegebene Bedeutung haben,
zunächst mit Acylierungsmitteln umsetzt, anschließend die an der Aminogruppe acylierte Verbindung der Formel II mit Kaliumpermanganat in einem bei pH-Werten zwischen 4 und 10 gehaltenen Medium oxidiert und danach den Acylrest von der Aminogruppe entfernt.

2. Neue 6-Ethyl-3-amino-2,4-dihalogen-pyridine der Formel II

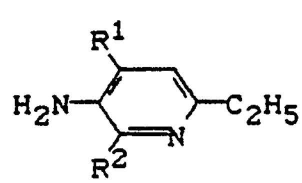

II

in welcher
$R^1$ und $R^2$ für Halogen stehen.

3. Verfahren zur Herstellung von 6-Ethyl-3-amino-2,4-dihalogenpyridinen der Formel II

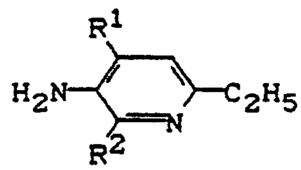

II

in welcher
$R^1$ und $R^2$ für Halogen stehen,
dadurch gekennzeichnet, daß man 6-Ethyl-3-nitro-2,4-dihalogenpyridine der Formel III

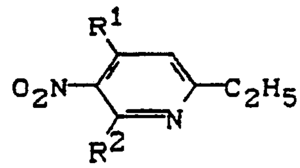

III

in welcher
$R^1$ und $R^2$ die oben angegebene Bedeutung haben
reduziert.

4. Neue 6-Ethyl-3-nitro-2,4-dihalogenpyridine der Formel III

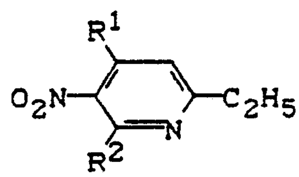

III

in welcher
$R^1$ und $R^2$ für Halogen stehen.

5. Verfahren zur Herstellung der neuen 6-Ethyl-3-nitro-2,4-dihalogen-pyridine der Formel III gemäß 4 (oben) dadurch gekennzeichnet, daß man 6-Ethyl-3-nitro-4-hydroxy-pyridon-(2)

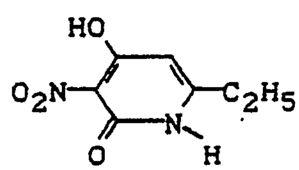

IV

mit Phosphoroxyhalogenid gegebenenfalls in Gegenwart von Säurebindemitteln umsetzt.

6. 6-Ethyl-3-nitro-4-hydroxy-pyridon-(2) der Formel IV ist neu.

7. Verfahren zur Herstellung von 6-Ethyl-3-nitro-4-hydroxy-pyridon-(2), dadurch gekennzeichnet, daß man 6-Ethyl-4-hydroxy-pyridon-(2) nitriert.

8. 6-Ethyl-4-hydroxy-pyridon-(2) der Formel (V) ist neu.

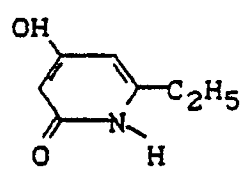

V

9. Verfahren zur Herstellung von 6-Ethyl-4-hydroxy-pyridon-(2), dadurch gekennzeichnet, daß man 6-Ethyl-4-hydroxy-3-carbalkoxypyridone der Formel VI

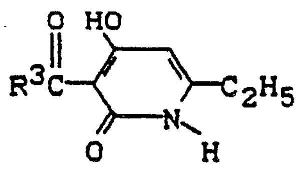

VI

in welcher
$R^3$ für OH oder $C_{1-3}$-Alkoxy steht,
in Gegenwart von Basen decarboxyliert.

10. Verwendung von 6-Acetyl-3-amino-2,4-dihalogenpyridinen der Formel I zur Herstellung von 6-Halogenacetyl-3-amino-2,4-dihalogenpyridinen der Formel VII

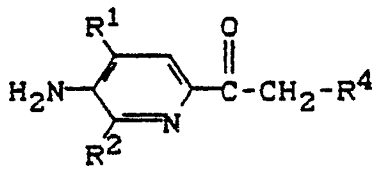

VII

in welcher
$R^1$, $R^2$ und $R^4$ unabhängig voneinander für Halogen stehen,
dadurch gekennzeichnet, daß man 6-Acetyl-3-amino-2,4-dihalogenpyridine der Formel I halogeniert.

Die 6-Halogenacetyl-3-amino-2,4-dihalogenpyridine können durch Reduktion der Carbonylgruppe und anschließende Umsetzung mit primären oder sekundären Aminen in die entsprechenden 3-Amino-2,4-dihalogen-pyridylethanolamine überführt werden. Diese dienen als Leistungsförderer bei Tieren (DE-OS 3 615 293).

Bevorzugt werden nach Verfahren 1 Verbindungen der Formel I hergestellt, in welcher $R^1$ und $R^2$ für Chlor oder Brom stehen.

Setzt man die Verfahren 1) 6-Ethyl-3-amino-2,4-dichlorpyridin als Verbindung der Formel II ein, lassen sich die einzelnen Stufen des Verfahrens durch folgende Formelschemata wiedergeben:

Stufe 1:

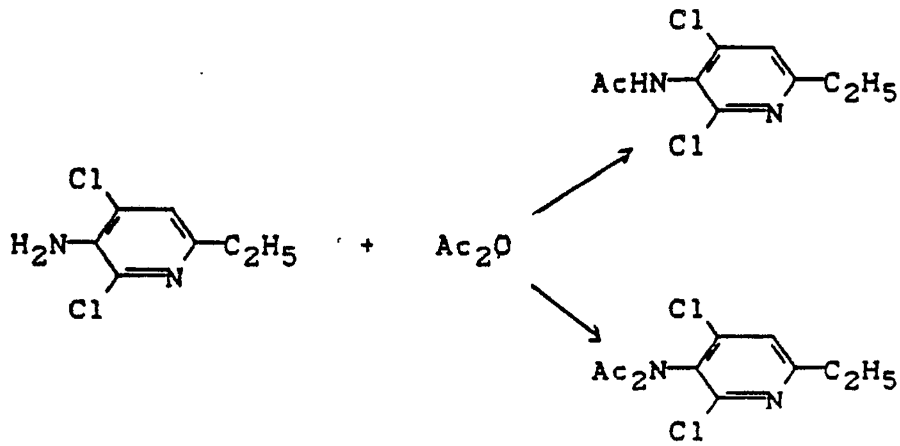


(Ac = Acetyl)

Stufe 2:

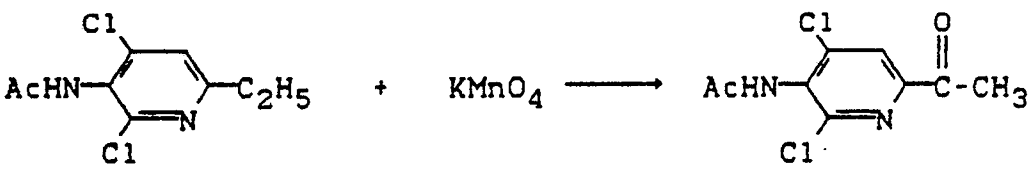


Stufe 3:

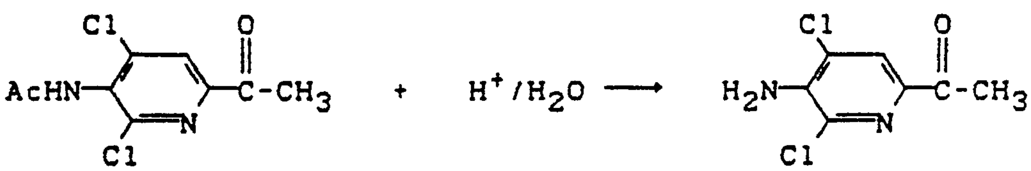


Die Verbindungen der Formel II sind neu. Ihre Herstellung wird weiter unten beschrieben.

Die Acylierung der 1. Stufe erfolgt mit Acetanhydrid, Acetylchlorid oder Keten. Bevorzugt wird in Gegenwart von Verdünnungsmitteln gearbeitet. Als solche seien genannt alle inerte organischen Lösungsmittel. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone wie Aceton, Methylethyl-, Methylisopropyl- und Methylisobutylketon, außerdem Ester wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon sowie Tetramethylensulfon und Hexamethylphosphorsäuretriamid.

Die Acylierung der 1. Stufe erfolgt bevorzugt in Gegenwart von basischen Katalysatoren oder Acylierungskatalysatoren. Als solche seien genannt: z.B. tertiäre Amine wie Pyridin, 4-Dimethylaminopyridin, Triethylamin, Triethylendiamin, Trimethylen-tetrahydropyrimidin; ferner Zinn-II- und Zinn-IV-Verbindungen wie Zinn-II-octoat oder Zinn-IV-chlorid. Die als Reaktionsbeschleuniger genannten tertiären Amine, wie z.B. Pyridin, können auch als Lösungsmittel verwendet werden.

Besonders bevorzugt ist Natriumacetat.

Die Reaktion wird bei 0-250 ° C, bevorzugt bei 50-150 ° C durchgeführt. Sie wird bei Normaldruck durchgeführt.

Auf 1 Mol Verbindung der Formel I werden 1-5 Mol, bevorzugt 1-2 Mol Acylierungsmittel, 1-5 Mol, bevorzugt 1-2 Mol basische Katalysatoren, gegebenenfalls 0,01-1 Mol Acylierungskatalysatoren eingesetzt.

Nach Beendigung der Reaktion wird das Reaktionsgemisch mit Wasser verdünnt und mit einem mit Wasser nicht mischbaren Lösungsmittel extrahiert. Das Extraktionsmittel wid abdestilliert. Man erhält so ein Gemisch der mono-bzw. diacetylierten Verbindungen der Formel II. Dieses Gemisch kann entweder direkt weiter eingesetzt werden oder zunächst durch Behandeln mit Basen in Gegenwart von Verdünnungsmitteln in die entsprechenden Monoacetylverbindungen überführt werden.

Die Überführung in die Monoacetylverbindung erfolgt in Gegenwart von Wasser, Alkoholen wie Methanol, Ethanol, i-Propanol, Aceton sowie Mischungen derselben.

Als Basen werden verwendet: Alkali- und Erdalkalihydroxide und -carbonate, tertiäre Amine wie Triethanolamin, 4-Dimethylaminopyridin.

Die Reaktion wird bei 0-150 ° C, bevorzugt bei 10-100 ° C und Normaldruck durchgeführt. Das Reaktionsgemisch wird mit Wasser versetzt, mit einem unpolaren Lösungsmittel extrahiert und das Lösungsmittel abdestilliert. Der Rückstand wird in der 2. Stufe weiterbearbeitet.

In der zweiten Stufe wird die acetylierte Verbindung der Formel II mit Kaliumpermanganat bei pH-Werten von 4-10 oxidiert.

Die Oxidation wird in Wasser oder Mischungen von Wasser mit wassermischbaren organischen Verdünnungsmitteln wie Aceton, t-Butanol, Pyridin, Acetonitril, Glykoldimethylether durchgeführt.

Der pH-Wert der Lösung wird mittels Puffersubstanzen auf 4-10, bevorzugt 5-8 eingestellt.

Als Puffersubstanzen dienen Alkalidihydrogenphosphate wie Natriumdihydrogenphosphat, Magnesiumnitrat bzw. das Gemische aus Magnesiumoxid und Salpetersäure, Magnesiumsulfat, Calciumsulfat, Alkali- und Erdalkalibicarbonate.

Die Reaktion kann auch durch ständige Zugabe von Säuren wie z.B. Essigsäure im gewünschten pH-Bereich gehalten werden.

Die Oxidation wird bei Temperaturen von 0 bis 70 ° C, bevorzugt bei 10- 30 ° C durchgeführt.

Es wird pro Mol Verbindung der Formel II 1,1-6 Mol Kaliumpermanganat und 1,1-6 Mol, bevorzugt 1,1-3 Mol Puffersubstanz eingesetzt.

Nach erfolgter Reaktion wird $MnO_2$ abfiltriert, das Lösungsmittel abdestilliert und in üblicher Weise aufgearbeitet.

In der dritten Stufe wird die Acetylgruppe in Gegenwart von Säuren abgespalten. Als Säuren dienen z.B. Salzsäure, Schwefelsäure, Bromwasserstoffsäure, Phosphorsäure, Methansulfonsäure, Benzolsulfonsäure oder stark saure Ionenaustauscher.

Die Reaktion wird in geeigneten Verdünnungsmitteln durchgeführt. Als solche dienen die bei der Durchführung der Stufe 2 genannten Verdünnungsmittel, z.B. Alkohole wie Methanol, Ethanol, i-Propanol, Ketone wie Aceton.

Es wird bei Temperaturen zwischen 0 und 150 ° C, bevorzugt 50-120 ° C und bei Normaldruck gearbeitet.

Setzt man bei Verfahren 3) 6-Ethyl-3-nitro-2,4-dibrompyridin als Verbindung der Formel III ein, läßt sich die Reaktion durch folgendes Formelschema wiedergeben:

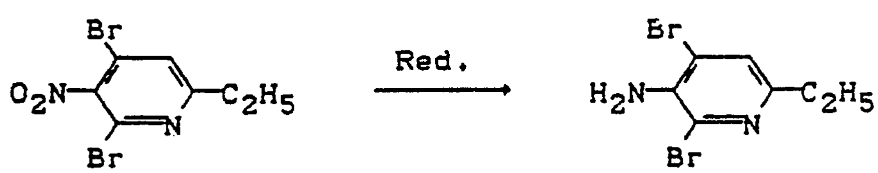


Die Nitropyridine der Formel II sind neu. Ihre Herstellung wird weiter unten (5) beschrieben.

Als Reduktionsmittel dienen bevorzugt Eisenfeilspäne, oder Zinn(II)chlorid in Gegenwart einer Säure.

Als Säuren dienen z.B. Halogenwasserstoffsäuren wie Salzsäure, Bromwasserstoffsäure, Eisessig. Es werden 0,3-10 Mol Säure auf ein Mol Verbindung der Formel III eingesetzt. Die Reduktion kann jedoch auch in der Säure als Verdünnungsmittel durchgeführt werden.

Es kann auch in Gegenwart anderer Verdünnungsmittel gearbeitet werden. Als solche dienen z.B. Alkohole wie Methanol, Ethanol, Butanol, Essigsäure, Wasser oder Mischungen dieser Verdünnungsmittel.

Das Reduktionsmittel wird im Überschuß eingesetzt. Es werden 6-15, bevorzugt 6-9 Reduktionsäquivalente Reduktionsmittel pro Mol der Verbindung III eingesetzt.

Die Reaktion wird zwischen 0 und 150 °C, bevorzugt 50-120 °C durchgeführt.

Die Aufarbeitung erfolgt, indem man nach beendeter Reduktion das Reaktionsgemisch filtriert, das Filtrat z.B. mit wäßriger Natronlauge alkalisch stellt und die Verbindung der Formel II in üblicher Weise extrahiert.

Verfahren 5) läßt sich durch folgendes Formelschema wiedergeben:

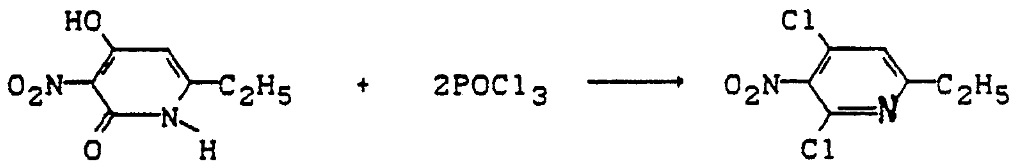

(IV)

6-Ethyl-3-nitro-4-hydroxy-pyridon-(2) der Formel IV ist neu. Seine Herstellung wird weiter unten beschrieben.

Verfahren 5) wird durchgeführt, indem man die Verbindung der Formel IV mit 2 bis 15 Mol, bevorzugt 2 bis 4 Mol des anorganischen Säurechlorids, gegebenenfalls in einem Verdünnungsmittel, behandelt.

Die Umsetzung wird bei Temperaturen von 20 °C bis 150 °C durchgeführt, es wird bevorzugt bei Normaldruck gearbeitet.

Die Umsetzung kann in Gegenwart von Säurebindemitteln erfolgen. Als solche seien bevorzugt tertiäre organische Amine genannt wie z.B. Triethylamin, Tripropylamin, Tributylamin, N,N-Dimethylanilin, Pyridin.

Als Verdünnungsmittel können alle inerten organischen Lösungsmittel verwendet werden. Hierzu zählen insbesondere aliphatische und aromatische, gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran, Dioxan, Nitrile, Ester.

Bevorzugt wird ohne Verdünnungsmittel gearbeitet.

Die Aufarbeitung erfolgt in an sich bekannter Weise durch Hydrolysieren des Halogenierungsmittels und Absaugen des Reaktionsproduktes bzw. Abdestillieren des Lösungsmittels.

Verfahren 7) läßt sich durch folgendes Formelschema wiedergeben:

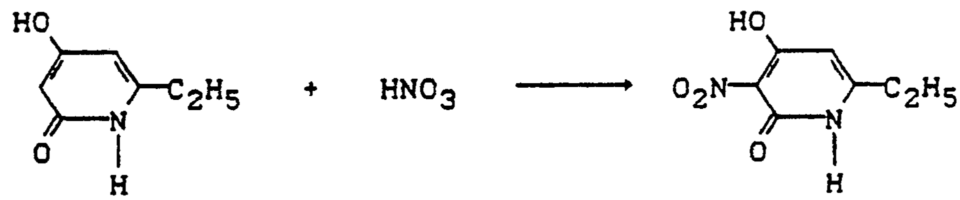

(V) (IV)

6-Ethyl-4-hydroxy-pyridon-(2) der Formel V ist neu. Seine Herstellung wird weiter unten beschrieben.

Die Reaktion wird in Salpetersäure oder in einem Gemisch aus Salpetersäure und Schwefelsäure als Nitrierungsmittel durchgeführt. Es werden mindestens 1-5 Mol Nitrierungsmittel pro Mol Verbindung der Formel V eingesetzt.

Es wird zwischen -10 und 150 °C, bevorzugt zwischen 0 und 100 °C sowie bei Normaldruck gearbeitet.

Das Reaktionsgemisch wird mit Wasser verdünnt und die Verbindung der Formel IV abfiltriert.

Verfahren 9) läßt sich durch folgendes Formelschema wiedergeben:

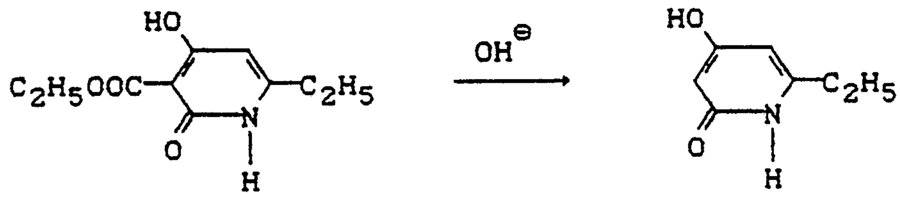

(VI) (V)

6-Ethyl-4-hydroxy-3-carbalkoxy-pyridone der Formel VI sind neu. Bevorzugt sind Verbindungen der Formel VI, in welcher $R^3$ für Methyl oder Ethyl steht.

Die Decarboxylierung zu Verbindungen der Formel V wird in Gegenwart wäßriger Basen wie Alkali- oder Erdalkalihydroxiden durchgeführt. Es werden 2-10, bevorzugt 2-5 Mol Base pro Mol der Verbindung der Formel VI eingesetzt. Es wird zwischen 20 und 200 °C, bevorzugt zwischen 50 und 150 °C gearbeitet.

Nach Beendigung der Reaktion wird das Gemisch schwach angesäuert und die Verbindung der Formel V abfiltriert.

Die Verbindungen der Formel VI können hergestellt werden, indem man Enamine der Formel VIII

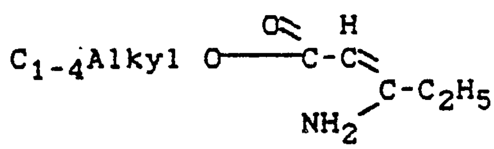

VIII

in welcher
$C_{1-4}$-Alkyl bevorzugt für Methyl oder Ethyl steht,
mit Malonestern der Formel IX

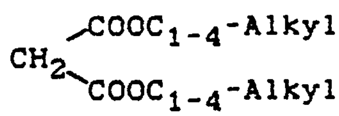

IX

in welcher
$C_{1-4}$-Alkyl bevorzugt für Methyl oder Ethyl steht,
in Gegenwart von Basen umsetzt.

Die Verbindungen der Formel VIII und IX werden im molaren Verhältnis von etwa 1:1 eingesetzt.

Als Basen dienen Alkali- und Erdalkalialkoholate wie Natriummethylat oder Kalium-t-butylat.

Die Basen werden im Verhältnis 1:3, bevorzugt 1:1,5 pro Mol Malonester eingesetzt.

Als Verdünnungsmittel dienen Alkohols wie Methanol, Ethanol, i-Propanol, t-Butanol sowie inerte organische Lösungsmittel, insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, darüber hinaus Amide wie z.B. Dimethylformamid, Dimethylacetamid und N- Methylpyrrolidon sowie Dimethylsulfoxid, Tetramethylensulfon und Hexamethylphosphorsäuretriamid.

Wie bereits erwähnt (10) lassen sich die 6-Acetyl-3-amino-2,4-dihalogenpyridine der Formel I verwenden, um Pyridinethanolamine herzustellen.

Die dabei durchlaufenen Reaktionsstufen können durch folgende Formelschemata wiedergegeben werden:

10a

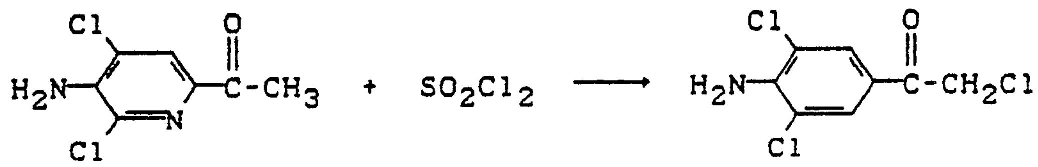

10b

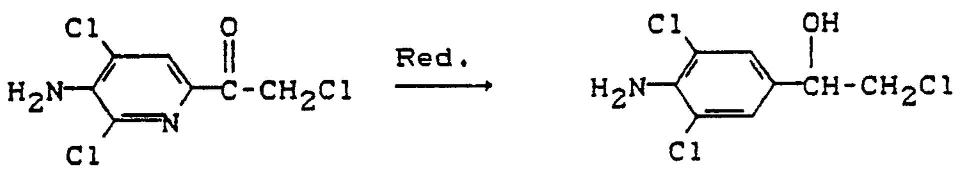


10c

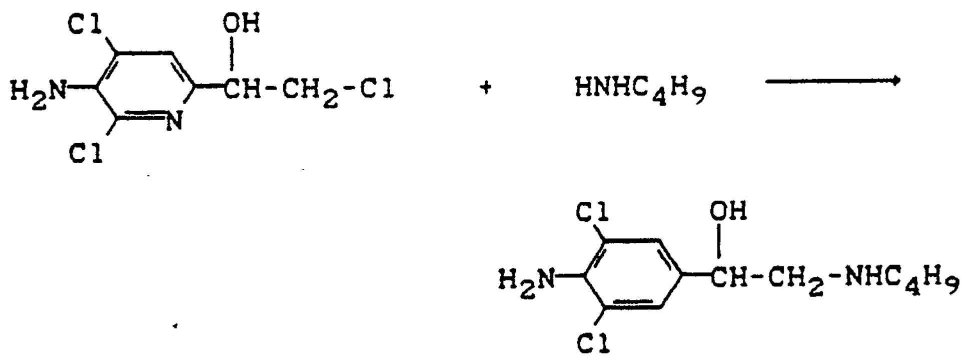

Als Halogenierungsmittel in Reaktionsstufe 10a wird bevorzugt Sulfurylchlorid eingesetzt.

Es wird 1-3, bevorzugt 1-1,5 Mol Halogenierungsmittel pro Mol der Verbindung der Formel I eingesetzt.

Es wird in Gegenwart von Verdünnungsmitteln gearbeitet. Als solche seien genannt: aliphatische, gegebenenfalls halogenierte Kohlenwasserstoffe wie z.B. Pentan, Hexan, Heptan, Ligroin, Cyclohexan, Methylenchlorid, Ethylenchlorid, Chloroform, Tetrachlorkohlenstoff, Alkohole wie Methanol, Ethanol, Ester wie Essigsäuremethyl- oder -ethylester sowie Mischungen dieser Verdünnungsmittel.

Es wird zwischen -20 und +150° C, bevorzugt zwischen 0 und 70° C und bei Normaldruck gearbeitet.

Reaktionsstufe 10b wird bevorzugt mit den Reduktionsmitteln Diboran oder komplexen Hydriden wie z.B. Natriumborhydrid oder Lithiumborhydrid durchgeführt.

Bei Stufe 10c werden bevorzugt folgende Amine eingesetzt:

Ammoniak, Methylamin, i-Propylamin, i-Butylamin, sec.-Butylamin, tert.-Butylamin, Cyclopentylamin, Cyclohexylamin, 1-Cyclohexylethylamin, Benzylamin, 2-Phenyl-ethyl bzw. -propylamin.

Stufe 10 kann mit oder ohne Verdünnungsmittel durchgeführt werden. Als Verdünnungsmittel seien genannt:

Insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, ferner Nitrile, wie z.B. Acetonitril und Propionitril, Benzonitril, Glutarsäurenitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Stufe 10c kann in Gegenwart von Katalysatoren wie z.B. Alkalihalogeniden, z.B. Natriumbromid, Kaliumiodid, quartären Ammoniumiodiden oder -bromiden durchgeführt werden.

Es wird bei Temperaturen von 25-250° C, bevorzugt 50-150° C gearbeitet.

Es wird bei Normaldruck oder unter erhöhtem Druck gearbeitet.

Es werden 2-10 Mol, bevorzugt 2-3 Mol Amin pro Mol Halogenethanolpyridin eingesetzt.
Die Aufarbeitung nach erfolgter Reaktion erfolgt in an sich bekannter Weise.

Beispiel 1 (Verfahren 1 (Stufe 1))

3-Acetamido-2,4-dichlor-6-ethylpyridin

7,8 g (40,8 mmol) 3-Amino-2,4-dichlor-6-ethyl-pyridin werden in einer Mischung von 40 ml Acetanhydrid und 4 g (49 mmol) wasserfreiem Natriumacetat zwei Stunden unter Rückfluß erhitzt. Anschließend wird im Vakuum eingedampft, der Rückstand zwischen verdünnter $NH_3$-Lösung und $CHCl_3$ verteilt, die organische Phase abgetrennt, mit $Na_2SO_4$ getrocknet und eingedampft. Der Rückstand (11,2 g) besteht aus einem Gemisch von mono- und diacetyliertem Amin. Er wird in 50 ml Methanol gelöst, 10 g gepulvertes $K_2CO_3$ zugegeben und eine Stunde bei Raumtemperatur gerührt. Anschließend wird eingedampft, zwischen Wasser und $CHCl_3$ verteilt, die organische Phase abgetrennt und mit 5 %iger wäßriger $NaH_2PO_4$-Lösung gewaschen. Nach Trocknung mit $Na_2SO_4$ wird eingedampft. Der Rückstand wird mit Ether verrührt und abgesaugt. Ausbeute 7,7 g (80,9 % der Theorie) Schmelzpunkt: 142° C.

Beispiel 2 (Verfahren 1 (Stufe 2))

6-Acetyl-3-acetamido-2,4-dichlorpyridin

125,7 g (0,49 mol) $Mg(NO_3)_2 \bullet 6H_2O$ werden in einem 3 1-Dreihalskolben vorgelegt und in 410 ml Wasser gelöst. Dazu werden 34,4 g (0,15 mol) 3-Acetamido-2,4-dichlor-6-ethylpyridin, gelöst in 410 ml Aceton, gegeben und anschließend noch 370 ml Wasser und 55,5 g (0,35 mol) $KMnO_4$ zugefügt. Die Mischung wird bei 26° C 18 Stunden gerührt. Danach wird abgesaugt, der Filterkuchen sorgfältig mit Aceton gewaschen. Das Filtrat wird. eingedampft und zwischen Dichlormethan und Wasser verteilt. Die organische Phase wird abgetrennt, mit $Na_2SO_4$ getrocknet und eingedampft.
Ausbeute: 24,5 g, Schmelzpunkt: 133-7° C
Gehalt nach GC/MS:
41 % Produkt
58 % Edukt.

Beispiel 3 (Verfahren 1 (Stufe 3))

3-Amino-6-acetyl-2,4-dichlorpyridin

43,3 g einer Mischung von 41 % 6-Acetyl-3-acetamido-2,4-dichlorpyridin und 59 % 3-Acetamido-2,4-dichlor-6-ethylpyridin werden in 345 ml Methanol gelöst und dann mit 172,4 ml 36 %iger wäßriger HCl versetzt. Die Mischung wird 2 1/2 Stunden unter Rückfluß erhitzt. Anschließend werden 900 ml Wasser zugegeben, mit einem Eisbad abgekühlt. Der Niederschlag wird abgesaugt und mit Wasser gewaschen.
Ausbeute: 14,47 g (98 % der Theorie berechnet auf Gehalt des eingesetzten Edukts)
Schmelzpunkt: 162° C

Das Filtrat wird mit Natronlauge alkalisch gestellt und mit Dichlormethan extrahiert. Nach Eindampfen verbleiben 20 g (95,5 % der Theorie bezogen auf Gehalt des eingesetzten Edukts) an 3-Amino-2,4-dichlor-6-ethylpyridin.

Beispiel 4 (Verfahren 3)

3-Amino-2,4-dichlor-6-ethylpyridin

9 g (40,7 mmol) 2,4-Dichlor-6-ethyl-3-nitropyridin werden in eine Lösung von 33 g (0,146 mol) $SnCl_2 \bullet 2H_2O$ in 100 ml 36 %iger HCl eingetragen und 30 Minuten unter Rückfluß gekocht. Danach wird abgekühlt und in 400 ml 12 %ige NaOH unter Kühlung eingerührt. Es wird dreimal mit je 150 ml Dichlormethan extrahiert, der Extrakt mit $Na_2SO_4$ getrocknet und eingedampft.
Ausbeute: 7,8 g (quantitativ)

Beispiel 5 (Verfahren 5)

2,4-Dichlor-6-ethyl-3-nitropyridin

58 g (0,315 mol) 6-Ethyl-4-hydroxy-3-nitro-1H-2-pyridon werden in 500 ml $POCl_3$ eingetragen und 16 Stunden bei 77° C gerührt. Anschließend wird im Vakuum eingedampft, der Rückstand mit Eis hydrolysiert, mit NaOH neutralisiert und dreimal mit je 300 ml $CHCl_3$ extrahiert. Der Extrakt wird mit 5 %iger $NaH_2PO_4$-Lösung gewaschen, mit $Na_2SO_4$ getrocknet und eingedampft. Der Rückstand wird mit Dichlormethan über Kieselgel filtriert.
Ausbeute: 47 g (67,5 % der Theorie) gelbes Öl.

Beispiel 6 (Verfahren 7)

6-Ethyl-4-hydroxy-3-nitro-1-H-pyridon

20 g (0,145 mol) 6-Ethyl-4-hydroxy-1-H-2-pyridon werden in 60 ml 65 %iger $HNO_3$ suspendiert und auf 65° C hochgeheizt. Die stark exotherme Reaktion wird durch Kühlung mit Eis unter Kontrolle gehalten. Nach Abklingen der Reaktion werden 120 ml Wasser zugegeben und mit Eis gekühlt. Nach 30 Minuten wird abgesaugt und getrocknet.
Ausbeute: 13,7 g (51 % der Theorie)
Schmelzpunkt: 185° C.

Beispiel 7 (Verfahren 9)

6-Ethyl-4-hydroxy-1H-2-pyridon

119 g (0,564 mol) 3-Carbethoxy-6-ethyl-4-hydroxy-1H-2-pyridon werden in einer Mischung von 1 l Wasser mit 155 g (2,77 mol) KOH zwei Stunden unter Rückfluß erhitzt. nach Abkühlung wird mit konzentrierter HCl auf pH 4 gestellt, wieder abgekühlt und abgesaugt.
Ausbeute: 74,7 g (95 % der Theorie)
Schmelzpunkt: 274° C.

Beispiel 8 (Herstellung der Verbindungen der Formel VI)

3-Carbethoxy-6-ethyl-4-hydroxy-1H-2-pyridon

Zu einer Lösung von 259,2 g (0,8 mol) 21 %iger Natriummethylat-Lösung in Methanol und 130 g Ethanol werden 113,6 g (0,88 mol) 3-Amino-2-pentansäuremethylester und 128 g (0,8 mol) Malonsäurediethylester gegeben. Nach Zugabe von 800 ml 1,2-Dichlorbenzol wird im Verlaufe von einer Stunde ein Gemisch von Methanol und Ethanol abdestilliert und die verbleibende Suspension zwei Stunden auf 160° C erhitzt. Nach dem Abkühlen werden 600 ml Wasser zugesetzt, die organische Phase abgetrennt, mit Wasser extrahiert und dann verworfen. Die vereinigten wäßrigen Phasen werden mit Ether gewaschen, die wäßrige Phase abgetrennt, mit Salzsäure angesäuert und abgesaugt.
Ausbeute: 118,9 g (70,5 % der Theorie)

Schmelzpunkt: 189 °C.

Beispiel 9 (Verfahren 10 (Stufe a))

3-Amino-6-chloracetyl-2,4-dichlorpyridin

14 g (68,3 mmol) 6-Acetyl-3-amino-2,4-dichlorpyridin werden in 70 ml trockenem $CHCl_3$ gelöst und dann eine Lösung von 5,5 ml (68,4 mmol) $SO_2Cl_2$ in 70 ml trochenem $CHCl_3$ zugetropft und zwei Stunden bei 40 °C gerührt. Zur Aufarbeitung wird auf 250 ml $NaHCO_3$-Lösung gegossen, die organische Phase abgetrennt und mit 100 ml $CHCl_3$ nachextrahiert. Die vereinigten organischen Phasen werden mit $Na_2SO_4$ getrocknet und eingedampft.
Ausbeute: 16 g, nicht weiter gereinigt.
Schmelzpunkt: 142-4 °C.

Beispiel 10 (Verfahren 10 (Stufe b))

2-Chlor-1-(3-amino-2,4-dichlor-6-pyridyl)-ethanol

16 g (66,8 mmol) 3-Amino-6-chloracetyl-2,4-dichlorpyridin werden in 150 ml Methanol suspendiert, auf 0 °C gekühlt und 2,78 g (73,5 mmol) $NaBH_4$ eingetragen. Es wird 15 Minuten bei 0 °C gerührt, dann auf 300 ml 5 %ige $NaH_2PO_4$0Lösung gegossen und 15 Minuten gerührt. Dann wird mit $CHCl_3$ erschöpfend extrahiert. Nach Trocknung mit $Na_2SO_4$ wird eingedampft und zur Reinigung über Kieselgel mit $CH_2Cl_2$/Essigester als Laufmittel chromatographiert.
Ausbeute: 13,1 g (81 % der Theorie)
Schmelzpunkt: 74 °C.

Beispiel 11 (Verfahren 10 (Stufe c))

2-N-tert.-Butylamino-1-(3-amino-2,4-dichlor-6-pyridyl)-ethanol

3 g (12,4 mmol) 2-Chlor-1-(3-amino-2,4-dichlor-6-pyridyl)-ethanol und 60 mg Tetrabutylammoniumiodid werden zusammen mit 24 ml tert.-Butylamin 12 Stunden auf 100 °C erhitzt. Zur Aufarbeitung wird eingedampft, der Rückstand zwischen 200 ml 5 %iger $NaH_2PO_4$-Lösung und 200 ml Ether verteilt. Die organische Phase wird verworfen, die wäßrige Phase mit NaOH alkalisch gestellt und mit Dichlormethan extrahiert. Nach Trocknung mit $Na_2SO_4$ wird eingedampft, der Rückstand in 10 ml Ether gelöst und bis zur leichten Trübung Petrolether zugegeben. Zur Vervollständigung der Kristallisation wird über Nacht stehen gelassen, dann abgesaugt und mit Petrolether nachgewaschen.
Ausbeute: 1 g(30 % der Theorie)
Schmelzpunkt: 124 °C.

**Ansprüche**

1. Verfahren zur Herstellung von 6-Acetyl-3-amino-2,4-dihalogenpyridinen der Formel I

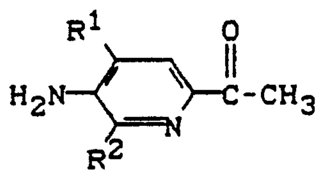

I

in welcher
$R^1$ und $R^2$ für Halogen stehen,
dadurch gekennzeichnet, daß man 6-Ethyl-3-amino-2,4-dihalogenpyridine der Formel II

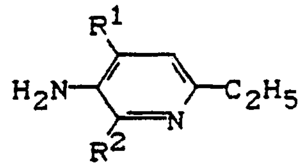

II

in welcher
$R^1$ und $R^2$ die oben angegebene Bedeutung haben
zunächst mit Acylierungsmitteln umsetzt, anschließend die an der Aminogruppe acylierte Verbindung der Formel II mit Kaliumpermanganat in einem bei pH-Werten zwischen 4 und 10 gehaltenen Medium oxidiert und danach den Acylrest von der Aminogruppe entfernt.

2. 6-Ethyl-3-amino-2,4-dihalogen-pyridine der Formel II

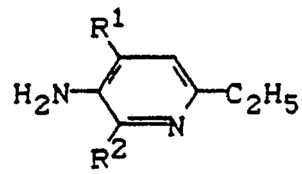

II

in welcher
$R^1$ und $R^2$ für Halogen stehen.

3. Verfahren zur Herstellung von 6-Ethyl-3-amino-2,4-dihalogenpyridinen der Formel II

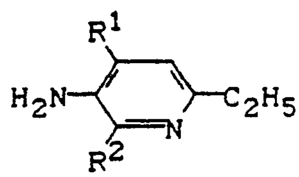

II

in welcher
$R^1$ und $R^2$ für Halogen stehen,
dadurch gekennzeichnet, daß man 6-Ethyl-3-nitro-2,4-dihalogenpyridine der Formel III

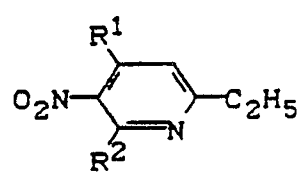

III

in welcher
$R^1$ und $R^2$ die oben angegebene Bedeutung haben,
reduziert.

4. 6-Ethyl-3-nitro-2,4-dihalogenpyridine der Formel III

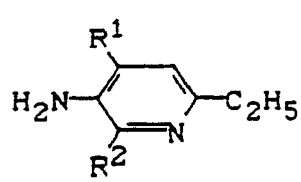

III

in welcher
$R^1$ und $R^2$ für Halogen stehen.

5. Verfahren zur Herstellung der neuen 6-Ethyl-3-nitro-2,4-dihalogen-pyridine der Formel III gemäß Anspruch 4 dadurch gekennzeichnet, daß man 6-Ethyl-3-nitro-4-hydroxy-pyridon-(2)

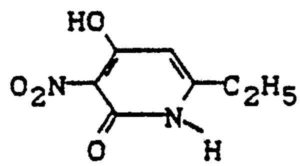

IV

mit Phosphoroxyhalogenid gegebenenfalls in Gegenwart von Säurebindemitteln umsetzt.

6. 6-Ethyl-3-nitro-4-hydroxy-pyridon-(2).

7. Verfahren zur Herstellung von 6-Ethyl-3-nitro-4-hydroxy-pyridon-(2), dadurch gekennzeichnet, daß man 6-Ethyl-4-hydroxy-pyridon-(2) nitriert.

8. 6-Ethyl-4-hydroxy-pyridon-(2).

9. Verfahren zur Herstellung von 6-Ethyl-4-hydroxy-pyridon-(2), dadurch gekennzeichnet, daß man 6-Ethyl-4-hydroxy-3-carbalkoxypyridone der Formel VI

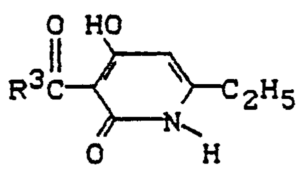

VI

in Gegenwart von Basen decarboxyliert.

10. Verwendung von 6-Acetyl-3-amino-2,4-dihalogenpyridinen der Formel I gemäß Anspruch 1 zur Herstellung von 6-Halogenacetyl-3-amino-2,4-dihalogenpyridinen der Formel VII

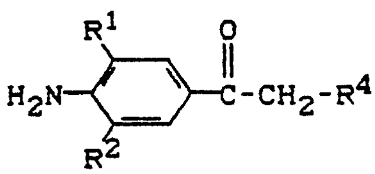

VII

in welcher
$R^1$, $R^2$ und $R^4$ unabhängig voneinander für Halogen stehen,
dadurch gekennzeichnet, daß man die 6-Acetyl-3-amino-2,4-dihalogenpyridine der Formel I halogeniert.